# EUROPEAN PATENT SPECIFICATION

(11) **EP 3 808 305 B1**
(45) Date of publication and mention of the grant of the patent: **06.11.2024**
(21) Application number: 19736766.7
(22) Date of filing: 03.06.2019
(51) Int. Cl.: A61B 90/14, A61G 13/02, A61G 13/10

(54) **REFERENCE NAVIGATION EQUIPMENT IN ROBOTIC SURGERY ASSISTED BY STEREOTAXIC NAVIGATION OF THE ORGANS AND SOFT PARTS OF A PATIENT'S PELVIC AREA**
REFERENZNAVIGATIONSGERÄT IN DER ROBOTERCHIRURGIE GESTÜTZT DURCH STEREOTAKTISCHE NAVIGATION DER ORGANE UND WEICHTEILE EINES BECKENBEREICHS EINES PATIENTEN
ÉQUIPEMENT DE RÉFÉRENCE DE NAVIGATION EN CHIRURGIE ROBOTIQUE ASSISTÉE AU MOYEN DE LA NAVIGATION STÉRÉOTAXIQUE DES ORGANES ET DES PARTIES MOLLES DE LA ZONE PELVIQUE D'UN PATIENT

(30) Priority: 15.06.2018 ES 201830919 U
(43) Date of publication of application: 21.04.2021
(73) Proprietor: Fundación Instituto de Investigación Marqués de Valdecilla (IDIVAL), 39011 Santander (Cantabria) (ES); Servicio Cántabro de Salud, 39011 Santander (Cantabria) (ES)
(72) Inventor: GÓMEZ RUIZ, Marcos, 39011 Santander - Cantabria (ES); CALLEJA IGLESIAS, Alberto, 39011 Santander - Cantabria (ES); ALONSO HORGA, Felipe, 39011 Santander - Cantabria (ES); MARTÍN LÁEZ, Rubén, 39011 Santander - Cantabria (ES)
(74) Representative: Pons
(86) International application number: PCT/ES2019/070373
(87) International publication number: WO 2019/238987

(56) References cited:
- WO-A1-02/064042
- WO-A1-2013/192598
- WO-A1-2013/192598
- US-A- 5 728 106
- US-A- 5 728 106
- US-A- 6 162 222
- US-A1- 2004 260 311
- US-A1- 2004 260 311
- US-A1- 2006 142 657
- US-A1- 2006 142 657
- US-A1- 2008 201 016
- US-A1- 2008 201 016
- US-A1- 2012 186 588
- US-A1- 2012 186 588

## Description

### OBJECT OF THE INVENTION

The present invention falls within the technical field of medical equipment for surgery. More specifically, it proposes equipment for navigation reference in assisted robotic surgery by means of stereotactic navigation of the organs and soft portions of the pelvic area of a patient. This equipment enables coordinates to be fixed with respect to a reference frame to be attached to any point of the patient's anatomy in the pelvic area.

### BACKGROUND OF THE INVENTION

Until now, surgery has been the only treatment for rectal cancer with demonstrated curative potential. In the evolution of the surgical treatment of this disease there is a key milestone which is the concept introduced by Heald of the need for total mesorectal excision (TME) in order to achieve a suitable oncological treatment. The suitable execution of this surgical technique, which is essential for achieving the correct oncological results, is a large technical need.

The difficulty of the resection of the rectum with TME is determined by the anatomic ratios thereof. The rectum is located in a narrow and angled musculoskeletal tunnel with an abdominal inlet mouth and another outlet one which is the anal sphincter or, surgically, the perineum. Inside that tunnel and closely related to the rectum is a portion of the male genital apparatus, bladder, seminal vesicles, prostate and urethra or the vagina and a bundle of nerves which will provide bowel, genital and urinary functionality.

This gives the rectal resection great technical difficulty due to problems related to visualisation and dissection, and the conventional surgical techniques imply a series of complications derived from this difficulty. This makes it so the surgeon factor is a crucial element in the clinical results. Overcoming these challenges is only possible by applying the best knowledge of the disease, best imaging techniques and, above all, the development of technologies that help the surgeon solve the barriers derived from the anatomic placement of the rectum.

The advances in digital technology are being applied in surgery with great success. Currently, so-called digital operating rooms are already commonly used. In some disciplines, mainly in neurosurgery, 3D images obtained from the patient (computerised axial tomography, magnetic resonance, etc.) have been able to be applied successfully. With a development of imaging integration, by means of navigation software, this 3D technology can be used to change the manner of performing the surgical method. Preoperative planning of the surgery can be determined, performing "virtual surgery" and it also acts as support for taking surgical decisions in real time.

Like a GPS guides a driver along the selected route and updates the position of the vehicle on the map, the image-guided surgery system (navigator) facilitates planning and simulating the surgical approach for the surgeon. Likewise, it enables information to be obtained about the location and progression of the surgical instruments to the lesion and preserves the integrity of the adjacent anatomic structures preventing complications.

In the past few years, the increase in complexity and the need for surgeons to perform minimally invasive surgery have driven the advance of the intraoperative imaging techniques. In this manner, solutions have been developed such as multidimensional equipment, computerised axial tomography and intraoperative resonances. These systems enable the images used by the navigator to be updated at any time during the operation.

However, navigation in anatomic areas that are not stiff (skull, spine) is still a significant challenge. First, it is not always possible to fasten a reference to the anatomy of the patient close to the work area. Second, the organs and soft tissues can be moved, making the navigation lose accuracy. Finally, although there are universal adapters for navigating with certain instruments (aspirators, bipolar forceps, endoscope), the fastening thereof is not always easy and sometimes does not enable them to be used ergonomically.

For these reasons, it is of interest in the field of pelvic surgery, and of rectal cancer in particular, the development of a system which enables image-guided surgery.

From the state of the art, a few reference systems for surgery are known among which the one described in document WO02064042A1 is highlighted. It discloses a patient support system which is maintained in a certain position during pelvic surgeries. It comprises a fastening arc containing two arms, fastening means for fastening to the pelvis and a reference device on surgical equipment and another on the arc.

The most important technical problem associated with this system is that it is configured to be used in specific surgeries in bony portions of the pelvis or of the hip, located on the outside of the pelvis. This system cannot be used to perform robotic surgery of the organs and soft portions of the inside of the abdominal cavity and the pelvis.

In order to achieve navigation in surgery of soft portions of the pelvis we need to fasten to the patient with an accuracy level close to 1 mm. Any other accuracy level could easily give rise to lesions of large vessels or of other organs. The device described in WO02064042A1 does not have a system for securing to the patient which makes it possible to achieve and maintain a sufficient accuracy level, both in the process before the operation as well as during surgery itself.

Likewise, the design of the fastening arc does not enable the securing of the patient to be combined with elements which ensure sufficient space for the work of the arms of the robot on the pelvic or abdominal area.

Other positioning systems are also known, like the one described in document CN105662551A which discloses an auxiliary positioning structure which supports a surgical instrument which is in turn fastened to the body of the patient or the one disclosed in DE10012042C1 which is a device for calibrating the position of the pelvis on an operating table wherein it is locked in the desired position and which comprises rods which emit orientation signals in order to enable surgical navigation.

Documents CN102283689A and US5971997A are also reference systems. For example, document CN102283689A describes a guide device for hip surgery which comprises an arc connected to two bars and an arm for the anchoring thereof to the patient. Furthermore, document US5971997A proposes an apparatus for calibrating a surgical navigator which has an arc for securing the head in a fixed position by means of screws and which comprises a surgical instrument with emitters that, by means of detectors, enable the position relative to the surgical field to be known.

Document US2012/186588A1 refers to a system includes a patient support panel and at least one positioning/fixation component for releasable mounting on the patient support panel at a desired position to immobilize a portion of the patient's body. The patient support panel includes a pair of longitudinally extending side rails having a series of longitudinally spaced indexing apertures for receipt of a locking bar for mounting a positioning/fixation component thereon at a discrete index position. The at least one positioning component includes a pair of clamping mechanisms for releasably securing that component to the side rails at any longitudinal position along the side rails.

Document US57281 06 refers to a rigid frame that is positioned around the skull to define a multi-dimensional coordinate system. Multiple arms are coupled together with a support ring to increase the overall stiffness of the frame. The arms and the support ring are made from a plastic material that makes the frame appear transparent in CT and MRI images. Receptors provide a mounting base upon which the frame is repeatedly attached and detached from the skull at the same reproducible reference location.

Document US2006142657 refers to a surgical apparatus includes a surgical device, configured to be manipulated by a user to perform a procedure on a patient, and a computer system. The computer system is programmed to implement control parameters for controlling the surgical device to provide at least one of haptic guidance to the user and a limit on user manipulation of the surgical device, based on a relationship between an anatomy of the patient and at least one of a position, an orientation, a velocity, and an acceleration of a portion of the surgical device, and to adjust the control parameters in response to movement of the anatomy during the procedure.

### DESCRIPTION OF THE INVENTION

The present invention describes equipment for navigation reference in assisted robotic surgery by means of stereotactic navigation of the organs and soft portions of the pelvic area (abdominal cavity and pelvis).

The equipment comprises a structure which is modular and adaptable and with at least one fastening arc wherein at least one reference device is in a position visible by the navigator of the robot performing the surgery. At the same time, the structure is configured to be joined to the anatomy of the patient, leaving enough free space for the robotic arm to move suitably and perform all the necessary operations.

The object of the invention is to ensure that any point of the anatomy of the patient in the pelvic area has coordinates fixed with respect to a first reference device joined to the fastening arc. Moreover, the equipment comprises a second reference device configured to couple to the instrumentation of the robot, in a position also visible by the navigator. The combination of these two reference devices makes the navigation of the robot and the triangulation of the position thereof possible.

The geometry of the structure has been designed such that there are no collisions with the mobile elements of the robot during the surgery or with the structure of an O-Arm (scanning device) in the process of obtaining images of the anatomy of the patient by means of a scanner. It is manufactured almost entirely from titanium since it is radiotransparent, in order to prevent interference during the scanning of the patient.

The structure comprises a fastening arc which is joined to the operating table whereon the patient is placed and can be adjusted in inclination, with respect to said table, and in extension, meaning in height, measured with respect to the table.

It also comprises at least two arms with several degrees of freedom with respect to the fastening arc and that are configured to be joined to the patient through two adapters which are fastened to the pelvis. Preferably, said adapters are threaded pins intended to be joined to the iliac crests of the patient. Thus, it prevents the use of additional tools for the fastening of the equipment to the patient which implies an improvement with respect to solutions known from the state of the art.

In the upper portion of the fastening arc the first reference device is joined, assembled such that the position thereof can be varied with respect to the arc and therefore with respect to the table (and to the patient lying thereon).

### DESCRIPTION OF THE DRAWINGS

As a complement to the description provided below, and for the purpose of helping to make the features of the invention more readily understandable, in accordance with a practical preferred embodiment thereof, said description is accompanied by a set of drawings which, by way of illustration and not limitation, represent the following:
Figure 1 shows a perspective view of the fastening arc, the arms and the first reference device of the equipment for navigation reference in assisted robotic surgery by means of stereotactic navigation of the organs and soft portions of the pelvic area of a patient.
Figure 2 shows a front view of the fastening arc, the arms and the first reference device.
Figure 3 shows a top view of the fastening arc, the arms and the first reference device.
Figure 4 shows a perspective view of the equipment with the components thereof in a position wherein the adapters of the arms are joined to the hip of a patient.
Figure 5 shows a lateral view of the fastening arc, the arms and the first reference device in the position of Figure 4 wherein the adapters of the arms are joined to the hip of a patient.
Figure 6 shows a perspective view of the equipment and of the robotic arm wherein the second reference device is found.
Figure 7 shows a zoomed-in view of one of the arms with the corresponding adapter thereof and the movements of the elements of said arms have been represented with arrows.

### PREFERRED EMBODIMENT OF THE INVENTION

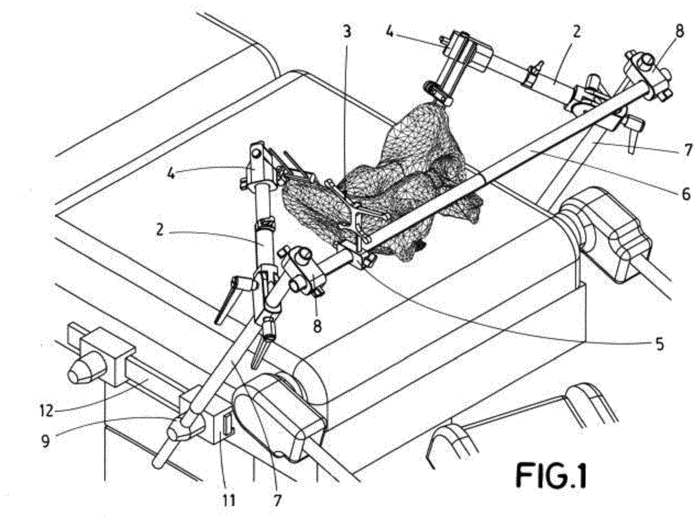

An exemplary embodiment of the invention is described below with help from Figures 1 to 7.

More specifically, it proposes equipment for navigation reference in assisted robotic surgery by means of stereotactic navigation of the organs and soft portions of the pelvic area of a patient with surgical instrumentation installed in a robot.

Figure 1 shows a perspective view of the equipment of the invention joined to a table whereon a patient to be operated on is placed.

The equipment comprises a fastening arc (1) configured to be joined to a surgical table whereon the patient is placed, at least two arms (2) joined to the fastening arc (1) configured to be joined to the patient and reference marks by means of which the navigation of the robot is controlled.

The most important features of the present invention are what enable the correct movement of the robot without there being mishaps with the equipment, which also does not interfere with the scanning equipment and which is completely adaptable to the measurements of the table and of the patient.

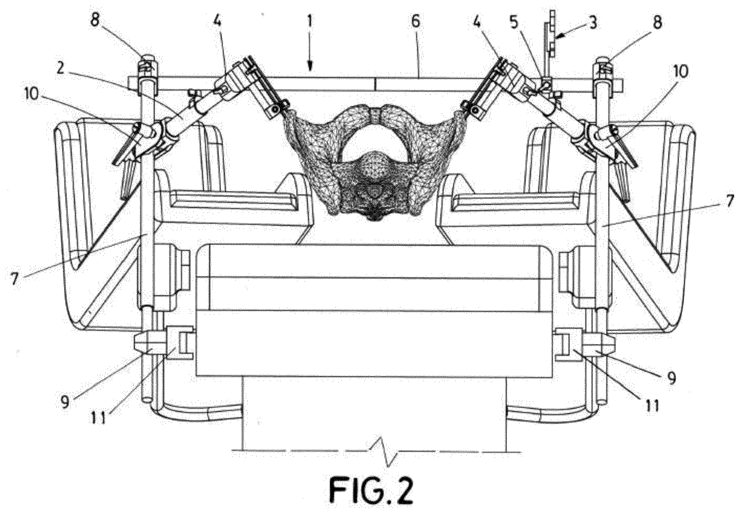

To do so, the fastening arc comprises, as seen in Figure 2, an upper bar (6) contained in a plane parallel to the surface of the table, two lateral bars (7), joined to the upper bar (6) forming an arc over the table and first joining means (8) and second joining means (9).

The first joining means (8) are linked to the lateral bars (7) and to the upper bar (6) in order to join them. They have the possibility of movement along the upper bar (6) until the fastening thereof in a position adapted to the width of the table wherein the equipment is fastened and they have the possibility of movement along the lateral bars (7) until the fastening thereof in a point corresponding to a height adapted to the position of the pelvis of the patient on the table.

The second joining means (9) are configured to join the lateral bars (7) to the table with the possibility of tilting of the lateral bars (7) around the axial axis of said second joining means (9), in order to regulate the inclination of the lateral bars (7) with respect to the table.

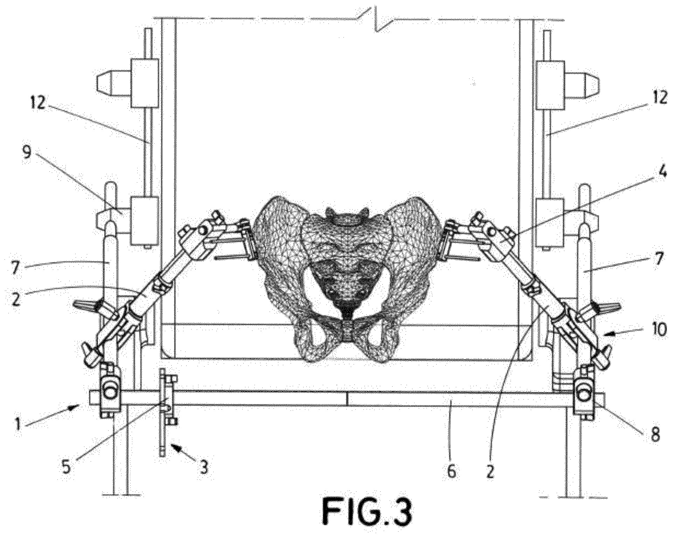

It can also be seen, for example in Figure 3, that the arms (2) are joined to the lateral bars (7) of the fastening arc (1) by means of third fastening means (10) which have the possibility of movement along the lateral bars (7), with the possibility of rotation with respect to said lateral bars (7). Furthermore, said third fastening means (10) comprise a ball joint configured to enable the tilting of the arms (2) with respect to the longitudinal axis of the lateral bars (7), and each arm comprises an adapter (4) on the free end thereof configured to be temporarily anchored to the patient. The joining of the adapters (4) to the pelvis of the patient just as it stays in the position wherein the equipment is used can be seen for example in Figures 1 to 3.

The equipment also comprises a first reference device (3) comprising a mobile part (5) joined to the upper bar (6) with the possibility of movement along said upper bar (6). It also has the possibility of rotation around the upper bar (6) and comprises at least one first reference mark detectable by a navigator of the robot.

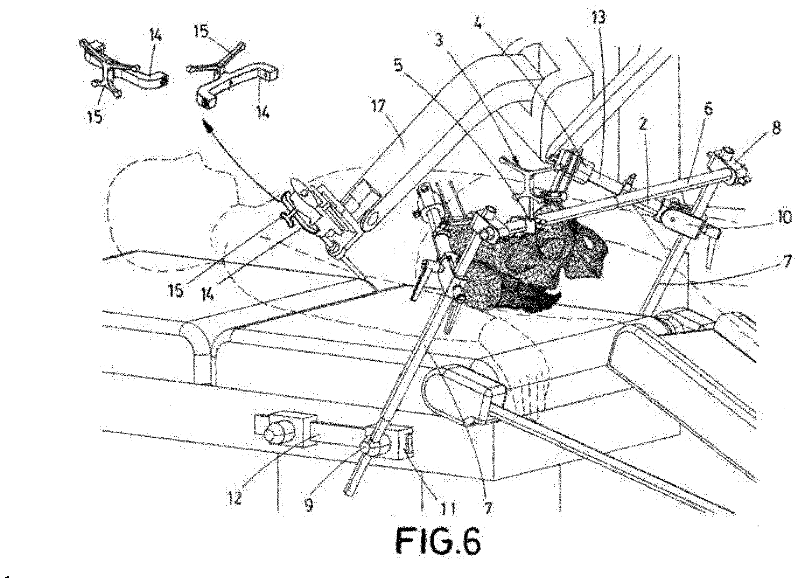

Likewise, the equipment comprises a second reference device (15) configured to be placed in the surgical instrumentation arranged in the robotic arm (17) of the robot by means of a part for fastening to the surgical instrumentatio (14). Said second reference device comprises at least one second reference mark detectable by the navigator. The first reference mark and the second reference mark enable the spatial position of the first reference device (3) with respect to the anatomy of the patient to be triangulated with the navigator. Figure 6 shows a zoomed-in view of the second reference device (15) and the part for fastening to the surgical instrumentation.

As described previously, an essential feature of the invention is that the equipment does not interfere with the scanning equipment. This is thanks to the fact that the fastening arc (1) and the arms (2), with the corresponding fastening means thereof, are made of a radiotransparent material. Preferably, said material is titanium.

Furthermore, in order to ensure a correct fastening of the adapters (4) to the anatomy of the patient, they comprise at least one threaded pin, a screw or another fastening element configured to be anchored to the patient.

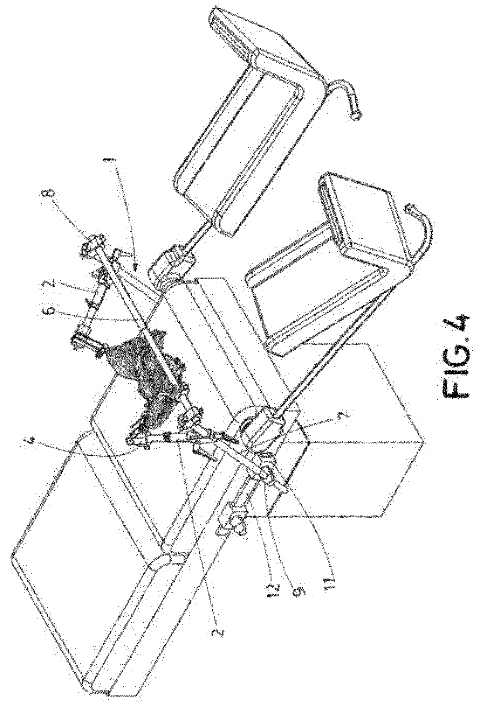

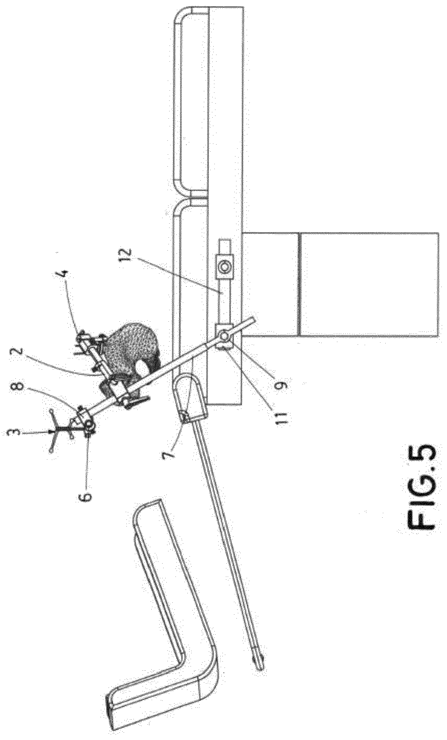

Figures 4 and 5 show views of the equipment or a portion of it, perspective and lateral views respectively, as it would be in the final position thereof, joined to the table and to the body of the patient. The high versatility of the equipment can be clearly seen which, thanks to the degrees of freedom of each of the components and fastening means thereof, can be adapted to different table measurements and patient anatomy sizes.

In an exemplary embodiment, like the one shown in said Figure 5, the second fastening means (9) are mounted on a slider (11) which has the possibility of movement on a rail (12) configured to be joined to the table. Thus, said second fastening means (9) have the possibility of movement on the rail (12).

Figure 6 shows a perspective view of the equipment and of the robotic arm (17) by means of which the operation is performed. In this case, the hip of the patient to which the adapters (4) of the arms (2) are joined can be seen. In order to facilitate the understanding of the invention, the patient is also represented, by dotted lines, in the position wherein they would be during the operation.

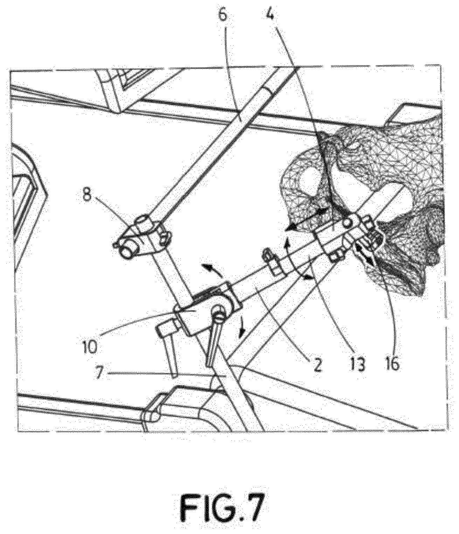

In an exemplary embodiment, the arms (2) can be extended by means of a bar (13) which has the possibility of movement from the lateral bars (7) to determine the length of the arms (2). This embodiment can be seen for example in Figure 7. Preferably, said bars (13) have the possibility of rotation with respect to the lateral bars (7).

Likewise, also preferably, the adapters (4) can be extended by means of a bolt (16) which has the possibility of movement from the arm (2) in order to determine the length of the adapter (4). In the case of the adapters (4) comprising at least one threaded pin, a screw or another fastening element configured to be anchored to the patient, it is arranged on one end of the bolt (16).

## Claims

1. Equipment for navigation reference in assisted robotic surgery by means of stereotactic navigation of the organs and soft portions of the pelvic area of a patient with surgical instrumentation installed in a robot, wherein the equipment comprises:
- a fastening arc (1), made of radiotransparent material, configured to be joined to a surgical table whereon the patient is placed and which in turn comprises:
- an upper bar (6) contained in a plane parallel to the surface of the table,
- two lateral bars (7) joined to the upper bar (6) by means of first joining means (8) and together forming an arc over the table; and
- second joining means (9) configured to join the lateral bars (7) to the table;
- two arms (2), made of radiotransparent material, joined at one end to the lateral bars (7) by means of third fastening means (10) which have the possibility of movement along the lateral bars (7), the possibility of rotation with respect to said lateral bars (7), and each arm (2) comprises an adapter (4) on the free end thereof configured to be temporarily anchored to the patient;
wherein
- the first joining means (8) have the possibility of movement along the lateral bars (7) and the upper bar (6);
- the lateral bars (7) have the possibility of tilting around the second joining means (9) in order to regulate the inclination of the lateral bars (7) with respect to the table;
- the third fastening means (10) comprise a ball joint configured to enable the tilting of the arms (2) with respect to the longitudinal axis of the lateral bars (7); and in that the equipment further comprises:
- a first reference device (3) comprising a mobile part (5) joined to the upper bar (6) with the possibility of movement along it and the possibility of rotation around said upper bar (6) and comprises at least one first reference mark detectable by a navigator;
- a second reference device (15) configured to be placed in the surgical instrumentation of the robot by means of a fastening device in the surgical instrumentation (14) and comprises at least one second reference mark detectable by the navigator for triangulating the spatial position of the first reference device (3).

2. The equipment according to claim 1, **characterised in that** the radiotransparent material is titanium.

3. The equipment according to claim 1, **characterised in that** the second fastening means (9) are mounted on a slider (11) which has the possibility of movement on a rail (12) configured to be joined to the table such that said second fastening means (9) have the possibility of movement on the rail (12).

4. The equipment according to claim 1, **characterised in that** the arms (2) can be extended by means of a bar (13) which has the possibility of movement from the lateral bars (7) to determine the length of the arms (2).

5. The equipment according to claim 4, **characterised in that** the bars (13) have the possibility of rotation with respect to the lateral bars (7).

6. The equipment according to claim 1, **characterised in that** the adapters (4) can be extended by means of a bolt (16) which has the possibility of movement from the arm (2) in order to determine the length of the adapter (4).

7. The equipment according to claim 6, **characterised in that** the adapters (4) comprise at least one threaded pin, a screw or another fastening element configured to be anchored to the patient.

8. The equipment according to claims 6 and 7, **characterised in that** the at least one threaded pin, a screw or another fastening element configured to be anchored to the patient is arranged on one end of the bolt (16).

## Patentansprüche

1. Apparatur zur Navigationsreferenz bei der assistierten Roboterchirurgie mittels stereotaktischer Navigation der Organe und Weichteile des Beckenbereichs eines Patienten mit in einem Roboter installiertem chirurgischem Instrumentarium, wobei die Apparatur Folgendes umfasst:
- einen Befestigungsbogen (1) aus strahlendurchlässigem Material, der dazu konfiguriert ist, mit einem Operationstisch verbunden zu werden, auf dem der Patient platziert wird und der wiederum Folgendes umfasst:
- eine obere Stange (6), die in einer Ebene parallel zur Tischoberfläche liegt,
- zwei seitliche Stangen (7), die mit der oberen Stange (6) mittels erster Verbindungsmittel (8) verbunden sind und zusammen einen Bogen über dem Tisch bilden; und
- zweite Verbindungsmittel (9), die dazu konfiguriert sind, die seitlichen Stangen (7) mit dem Tisch zu verbinden;
- zwei Arme (2) aus strahlendurchlässigem Material, die an einem Ende mit den seitlichen Stangen (7) mittels dritter Befestigungsmittel (10) verbunden sind, die sich entlang der seitlichen Stangen (7) bewegen können und in Bezug auf die seitlichen Stangen (7) drehen können, und wobei jeder Arm (2) an seinem freien Ende einen Adapter (4) umfasst, der dazu konfiguriert ist, vorübergehend am Patienten verankert zu werden;
wobei
- sich die ersten Verbindungsmittel (8) entlang der seitlichen Stangen (7) und der oberen Stange (6) bewegen können;
- die seitlichen Stangen (7) um die zweiten Verbindungsmittel (9) herum gekippt werden können, um die Neigung der seitlichen Stangen (7) in Bezug auf den Tisch zu regulieren;
- die dritten Befestigungsmittel (10) ein Kugelgelenk umfassen, das dazu konfiguriert ist, das Kippen der Arme (2) in Bezug auf die Längsachse der seitlichen Stangen (7) zu ermöglichen; und die Apparatur ferner Folgendes umfasst:
- eine erste Referenzvorrichtung (3), die einen beweglichen Teil (5) umfasst, der mit der oberen Stange (6) verbunden ist, sich entlang dieser bewegen kann und sich um die obere Stange (6) herum drehen kann und mindestens eine erste, von einem Navigator erkennbare Referenzmarkierung umfasst;
- eine zweite Referenzvorrichtung (15), die dazu konfiguriert ist, mittels einer Befestigungsvorrichtung im chirurgischen Instrumentarium im chirurgischen Instrumentarium (14) des Roboters platziert zu werden, und die mindestens eine zweite, vom Navigator erkennbare Referenzmarkierung zum Triangulieren der räumlichen Position der ersten Referenzvorrichtung (3) umfasst.

2. Apparatur gemäß Anspruch 1, **dadurch gekennzeichnet, dass** das strahlendurchlässige Material Titan ist.

3. Apparatur gemäß Anspruch 1, **dadurch gekennzeichnet, dass** die zweiten Befestigungsmittel (9) auf einem Schieber (11) angebracht sind, der sich auf einer Schiene (12) bewegen kann, die dazu konfiguriert ist, mit dem Tisch verbunden zu werden, sodass sich die zweiten Befestigungsmittel (9) auf der Schiene (12) bewegen können.

4. Apparatur gemäß Anspruch 1, **dadurch gekennzeichnet, dass** die Arme (2) mittels einer Stange (13), die sich von den seitlichen Stangen (7) aus bewegen kann, verlängert werden können, um die Länge der Arme (2) zu bestimmen.

5. Apparatur gemäß Anspruch 4, **dadurch gekennzeichnet, dass** sich die Stangen (13) in Bezug auf die seitlichen Stangen (7) drehen können.

6. Apparatur gemäß Anspruch 1, **dadurch gekennzeichnet, dass** die Adapter (4) mittels eines Bolzens (16), der sich vom Arm (2) aus bewegen kann, verlängert werden können, um die Länge der Adapter (4) zu bestimmen.

7. Apparatur gemäß Anspruch 6, **dadurch gekennzeichnet, dass** die Adapter (4) mindestens einen Gewindestift, eine Schraube oder ein anderes zur Verankerung am Patienten konfiguriertes Befestigungselement umfassen.

8. Apparatur gemäß Anspruch 6 und 7, **dadurch gekennzeichnet, dass** an einem Ende des Bolzens (16) der mindestens eine Gewindestift, eine Schraube oder ein anderes zur Verankerung am Patienten konfiguriertes Befestigungselement angeordnet ist.

## Revendications

1. Équipement de référence de navigation en chirurgie robotique assistée au moyen de la navigation stéréotaxique des organes et des parties molles de la zone pelvique d'un patient avec une instrumentation chirurgicale installée dans un robot, l'équipement comprenant :
- un arc de fixation (1), constitué de matériau radiotransparent, conçu pour être relié à une table chirurgicale sur laquelle est placé le patient et qui comprend à son tour :
- une barre supérieure (6) contenue dans un plan parallèle à la surface de la table,
- deux barres latérales (7) reliées à la barre supérieure (6) au moyen de premiers moyens de liaison (8) et formant ensemble un arc au-dessus de la table ; et
- des deuxièmes moyens de liaison (9) conçus pour relier les barres latérales (7) à la table ;
- deux bras (2), constitués de matériau radiotransparent, reliés à une extrémité aux barres latérales (7) au moyen de troisièmes moyens de fixation (10) qui ont la possibilité de se déplacer le long des barres latérales (7), la possibilité de tourner par rapport auxdites barres latérales (7) et chaque bras (2) comprenant un adaptateur (4) sur son extrémité libre conçu pour être ancré temporairement au patient ;
dans lequel
- les premiers moyens de liaison (8) ont la possibilité de se déplacer le long des barres latérales (7) et de la barre supérieure (6) ;
- les barres latérales (7) ont la possibilité de s'incliner autour des deuxièmes moyens de liaison (9) afin d'ajuster l'inclinaison des barres latérales (7) par rapport à la table ;
- les troisièmes moyens de fixation (10) comprennent une rotule conçue pour permettre l'inclinaison des bras (2) par rapport à l'axe longitudinal des barres latérales (7) ; et l'équipement comprenant en outre :
- un premier dispositif de référence (3) comprenant une partie mobile (5) reliée à la barre supérieure (6) avec la possibilité de se déplacer le long de celle-ci et la possibilité de tourner autour de ladite barre supérieure (6) et comprenant au moins un premier repère de référence détectable par un navigateur ;
- un second dispositif de référence (15) conçu pour être placé dans l'instrumentation chirurgicale du robot au moyen d'un dispositif de fixation dans l'instrumentation chirurgicale (14) et comprenant au moins un second repère de référence détectable par le navigateur pour trianguler la position spatiale du premier dispositif de référence (3).

2. Équipement selon la revendication 1, **caractérisé en ce que** le matériau radiotransparent est du titane.

3. Équipement selon la revendication 1, **caractérisé en ce que** les deuxièmes moyens de fixation (9) sont montés sur un coulisseau (11) qui a la possibilité de se déplacer sur un rail (12) conçu pour être relié à la table de telle sorte que lesdits deuxièmes moyens de fixation (9) ont la possibilité de se déplacer sur le rail (12).

4. Équipement selon la revendication 1, **caractérisé en ce que** les bras (2) peuvent être rallongés au moyen d'une barre (13) qui a la possibilité de se déplacer à partir des barres latérales (7) pour définir la longueur des bras (2).

5. Équipement selon la revendication 4, **caractérisé en ce que** les barres (13) ont la possibilité de tourner par rapport aux barres latérales (7).

6. Équipement selon la revendication 1, **caractérisé en ce que** les adaptateurs (4) peuvent être rallongés au moyen d'un boulon (16) qui a la possibilité de se déplacer à partir du bras (2) afin de définir la longueur de l'adaptateur (4).

7. Équipement selon la revendication 6, **caractérisé en ce que** les adaptateurs (4) comprennent au moins une tige filetée, une vis ou un autre élément de fixation conçu pour être ancré au patient.

8. Équipement selon les revendications 6 et 7, **caractérisé en ce que** l'au moins une tige filetée, une vis ou un autre élément de fixation conçu pour être ancré au patient est disposé sur une extrémité du boulon (16).
